# EUROPEAN PATENT SPECIFICATION

(11) **EP 1 444 356 B1**
(45) Date of publication and mention of the grant of the patent: **09.05.2012**
(21) Application number: 02795579.8
(22) Date of filing: 30.10.2002
(51) Int. Cl.: C07K 14/415, G01N 33/50, G01N 33/68

(54) **IDENTIFYING EPITOPES AND REDUCING THE ALLERGENICITY OF FOOD PROTEINS**
IDENTIFIKATION VON EPITOPEN UND ERNIEDRIGUNG DER ALLERGENITÄT VON NAHRUNGSMITTELPROTEINEN
IDENTIFICATION D'EPITOPES ET REDUCTION DE L'ALLERGENICITE DE PROTEINES ALIMENTAIRES

(30) Priority: 13.11.2001 US 350650 P
(43) Date of publication of application: 11.08.2004
(73) Proprietor: Danisco US Inc., Palo Alto, CA 94304 (US)
(72) Inventor: HARDING, Fiona, A, Santa Clara, CA 95050 (US); POWER, Scott, D., San Bruno, CA 94066 (US)
(74) Representative: Forrest, Graham Robert
(86) International application number: PCT/US2002/034879
(87) International publication number: WO 2003/042656

(56) References cited:
- WO-A-00/47610
- WO-A-00/52154
- WO-A-99/53038
- WO-A2-01/40281
- WO-A2-99/53038
- US-A1- 2003 041 354
- POULSEN L K: "In vivo and in vitro techniques to determine the biological activity of food allergens" JOURNAL OF CHROMATOGRAPHY. BIOMEDICAL APPLICATIONS, ELSEVIER, AMSTERDAM, NL, vol. 756, no. 1-2, 25 May 2001 (2001-05-25), pages 41-55, XP004249650 ISSN: 0378-4347
- VALENTA R ET AL: "INDUCTION OF SPECIFIC HISTAMINE RELEASE FROM BASOPHILS WITH PURIFIED NATURAL AND RECOMBINANT BIRCH POLLEN ALLERGENS" JOURNAL OF ALLERGY AND CLINICAL IMMUNOLOGY, MOSBY - YEARLY BOOK, INC, US, vol. 91, no. 1, PART 1, 1993, pages 88-97, XP009040150 ISSN: 0091-6749

## Description

### Field of the Invention

The present invention provides methods and composition concerning the identification and modification of allergenic proteins. More particularly, the present invention provides methods for the modification of epitopes of allergenic food proteins, as well as epitopes of potentially allergenic food proteins.

### Background of the Invention

Much work has been done to alleviate the problems of hypersensitivity in some individuals to proteins used in industrial, pharmaceutical and commercial applications. However, efforts are also required to identify and potentially reduce the allergenicity of food proteins.

Food allergy is reported to be the most common cause of anaphylaxis outside of the hospital (Kemp et al., Arch. Intern. Med., 1749 [1995]; and Yocum and Khan, Mayo Clinic Proc., 69:16-23 [1994]). Estimates indicate that from 5.4 million to 7 million Americans have food allergies (Sicherer et al., J. Allergy Clin. Immunol., 103:559-562 [2000]). Indeed, food allergies affect nearly everyone at some point. There are eight types of foods that are accountable for approximately 90% of all food-allergic reactions. The foods that most commonly cause anaphylaxis include peanuts, tree nuts, shellfish, fish, milk, soy, wheat and eggs. However, many other compounds have also been associated with food allergies. In addition, with the on-going development of various genetically-modified crops, there is concern that the modifications to the plants will result in allergic reactions in those who eat them.

Currently, there are no good methods of preventing and treating food allergies. Indeed, prevention of anaphylaxis and less serious allergic reactions typically involves avoidance of the foods that trigger the reaction. This is becoming increasingly difficult, as hidden ingredients may be included in food items, and product labels sometimes use alternative names for food ingredients that contain allergens. In addition, food allergies in children are often difficult to manage due to adults or children inadvertently providing the problem food to a food-sensitive child.

Treatment of food allergies is likewise problematic and people with food allergies need to always be prepared to treat their allergic reactions to food. Indeed, one study of children and adolescents with food allergies graphically demonstrates how important it is for those with food allergies to carry epinephrine with them at all times (Sampson et al., New Eng. J. Med., 327:380-384 [1992]). According to this study, 10 out of 13 fatal and near-fatal anaphylactic reactions to food occurred in public places and none of those who died had epinephrine with them. All of the cases who experienced such a reaction and received epinephrine before or within five minutes of developing severe symptoms survived. Thus, those who are highly sensitive to foods must carry epinephrine with them and know how to administer it to themselves. However, epinephrine use does not come without danger. Side effects of epinephrine include palpitations, tachycardia, sweating, nausea, vomiting, and respiratory difficulty. Thus, even after administration of epinephrine, emergency medical treatment is often needed, in order to deal with any respiratory or cardiac emergency. Following the administration of epinephrine, sequelae such as cardiac arrhythmias sometimes occur. In addition to epinephrine, some patients need to carry anti-histamines with them at all times as well. Those who are severely allergic to certain food items must be diligent in ensuring that they have their needed doses and means to administer the drug(s) to themselves. This is cumbersome and often impractical. Thus, what is needed are means to identify and modify food allergens such that their allergenicity is reduced. Indeed, the development of hypoallergenic food items would provide a great benefit to those who are sensitive to food allergens. WO 99/53038 and WO 00/52154 disclose methods for identifying and/or reducing the allergenicity of proteins, such as those derived from peanuts.

### Summary of the Invention

The present invention provides methods and composition concerning the identification and modification of allergenic proteins. More particularly, the present invention provides methods for the modification of epitopes of the allergenic food protein Ber e1, as well as potentially allergenic epitopes thereof.

The present specification discloses methods for identifying the allergenicity of a food protein comprising the steps of: obtaining a food protein; preparing a plurality of amino acid fragments of the food protein, such that each fragment overlaps in sequence with its contiguous fragments; contacting the amino acid fragments of the food protein with a solution comprising naïve human CD4+ T-cells and dendritic cells, wherein the dendritic cells have been differentiated; and identifying an epitope region with the amino acid fragments of the food protein, wherein the identifying step comprises measuring the ability of the epitope region to stimulate proliferation of the naïve human CD4+ T-cells. Eventually the dendritic cells and the CD4+ cells are obtained from a single blood source. The food protein may be selected from the group consisting of nuts, seafood, dairy products, soy, wheat, eggs, and genetically-modified food products. The epitope region comprises a T-cell epitope.

The present invention provides methods for reducing the allergenicity of the Ber e1 protein comprising the step of: modifying the epitope region of the allergenic food protein identified as Seq. ID 4, 6, or 7 to produce a food protein having reduced allergenicity. In some particularly preferred embodiments, the methods further comprise the step of determining the allergenicity of the food protein having reduced allergenicity.

In the preferred embodiments, the methods comprise, the additional steps of contacting a plurality of amino acid fragments of the food protein having reduced allergencity with a second solution comprising naïve human CD4+ T-cells and dendritic cells, wherein the dendritic cells have been differentiated; measuring the ability of the amino acid fragments of the mutant protease to stimulate proliferation of the naïve human CD4+ T-cells; and comparing the ability of the amino acid fragments of the food protein having reduced allergenicity to stimulate the proliferation of naïve human CD4+ T-cells with the ability of the amino acid fragments of the food allergen to stimulate the proliferation of naïve human CD4+ T-cells are provided. In still other embodiments, the dendritic cells and the CD4+ T-cells in the first and second solutions are obtained from a single blood source. In additional embodiments, the epitope is a T-cell epitope. The methods might also comprise the step of producing an expression vector comprising a nucleic acid sequence encoding the food allergen having reduced allergenicity. The methods might further comprise the step of transforming at least one host cell with the expression vector. In yet additional embodiments, the methods further comprise the steps of cultivating at least one host cell in a culture medium under conditions that promote the expression of the food protein having reduced allergenicity and recovering the food allergen having reduced allergenicity from the cell or the culture medium.

### Description of the Figures

Figure 1 illustrates the amino acid sequence of the mature protein of the Brazil nut seed storage protein known as allergen Ber e1 having SWISS-PROT accession number P04403. The mature protein Ber e1 protein (SEQ ID NO: 1) is made up of two small polypeptide chains: a heavy chain (73 amino acids) and a light chain (28 amino acids). The underlined sequences represent the epitope regions of the light and heavy chain proteins.
Figure 2 illustrates the protoxin amino acid sequence (SEQ ID NO: 2) of Cry 1Ab from *B. thuringiensis serovar aizawai* used to determine T-cell epitopes therein.
Figure 3 illustrates the protoxin amino acid sequence (SEQ ID NO: 3) of Cry 3Aa *from B. thuringiensis subsp. San-diego* used to determine T-cell epitopes therein.
Figure 4 illustrates the results (percent responders) for each of the 15-mer peptide strings of Ber e1 protein. The light chain is represented by peptides 1-6 and the heavy chain is represented by peptides 7-27.
Figure 5 illustrates the results (percent responders) for each of the 15-mer peptide strings of *Bacillus thuringienesis* cry protein Cry3Aa.
Figure 6 illustrates the results (percent responders) for each of the 15-mer peptide strings of the *Bacillus thuringienesis* cry protein Cry1Ab.
Figure 7 is an alignment of the amino acid sequences for Cry1Ab and Cry3Aa

### Definitions

Prior to providing a description of the invention, Applicants provide the following definitions.

As used herein, "allergenic food protein" refers to any food protein that is associated with causing an allergic reaction in humans and other animals. A "putative allergenic food protein" is a food protein that may be allergenic. A "food protein with reduced allergenicity" is a food protein that has been modified so as to be less allergenic (i.e., hypoallergenic) than the original, unmodified protein. It is intended that these terms encompass naturally-occurring food proteins, as well as those produced synthetically and/or using recombinant technology. The claimed invention only concerns the Ber e1 protein.
"Antigen presenting cell" and "APC" as used herein refer to cells of the immune system that present antigen on their surface in a manner that is recognizable by T-cells. Examples of antigen presenting cells are dendritic cells, interdigitating cells, activated B-cells and macrophages.
"T-cell proliferation" as used herein means the number of T-cells produced during the incubation of T-cells with the antigen presenting cells, with or without antigen. In some preferred embodiments, T-cell proliferation is assessed by measuring the amount of tritiated thymidine incorporated into the DNA of dividing cells.
"Baseline T-cell proliferation" as used herein means T-cell proliferation that is normally seen in an individual in response to exposure to antigen presenting cells in the absence of peptide or protein antigen. For the purposes herein, the baseline T-cell proliferation level was determined on a per sample basis for each individual as the proliferation of T-cells in response to antigen presenting cells in the absence of antigen.

The term "lymphoid" when used in reference to a cell line or a cell, means that the cell line or cell is derived from the lymphoid lineage and includes cells of both the B and the T lymphocyte lineages.

The terms "T lymphocyte" and "T-cell" as used herein encompass any cell within the T lymphocyte lineage from T-cell precursors (including Thy1 positive cells which have not rearranged the T cell receptor genes) to mature T cells (*i.e.*, single positive for either CD4 or CD8, surface TCR positive cells).

The terms "B lymphocyte" and "B-cell" encompasses any cell within the B-cell lineage from B-cell precursors, such as pre-B-cells (B220⁺ cells which have begun to rearrange Ig heavy chain genes), to mature B-cells and plasma cells.

As used herein, "CD4⁺ T-cell" and "CD4 T-cell" refer to helper T-cells, while "CD8⁺ T-cell" and CD8 T-cell" refer to cytotoxic T-cells.

As used herein "T-cell epitope" means a feature of a peptide or protein that is recognized by a T-cell receptor in the initiation of an immunologic response to the peptide comprising that antigen. Recognition of a T-cell epitope by a T-cell is generally believed to be via a mechanism wherein T-cells recognize peptide fragments of antigens which are bound to class I or class II Major Histocompatibility Complex (MHC) molecules expressed on antigen-presenting cells (*See e.g.*, Moeller (ed.), Immunol. Rev., 98:187 [1987]). In some embodiments of the present invention, the epitopes or epitopic fragments identified as described herein find use in the detection of antigen presenting cells having MHC molecules capable of binding and displaying the epitopes or fragments. In some embodiments, the epitopes/epitopic fragments further comprise a detectable label (*i.e.*, a marker) that facilitates the identification of cells that bind and/or display the epitope/epitopic fragment of interest.

As used herein, a "major epitope" is defined as a peptide sequence that induces responses in a percentage of the donor pool equal to approximately three times the background rate.

The term "recombinant DNA molecule" as used herein refers to a DNA molecule that is comprised of segments of DNA joined together by means of molecular biological techniques.

DNA molecules are said to have "5' ends" and "3' ends" because mononucleotides are reacted to make oligonucleotides in a manner such that the 5' phosphate of one mononucleotide pentose ring is attached to the 3' oxygen of its neighbor in one direction via a phosphodiester linkage. Therefore, an end of an oligonucleotides is referred to as the "5' end" if its 5' phosphate is not linked to the 3' oxygen of a mononucleotide pentose ring and as the "3' end" if its 3' oxygen is not linked to a 5' phosphate of a subsequent mononucleotide pentose ring. As used herein, a nucleic acid sequence, even if internal to a larger oligonucleotide, also may be said to have 5' and 3' ends. In either a linear or circular DNA molecule, discrete elements are referred to as being "upstream" or 5' of the "downstream" or 3' elements. This terminology reflects the fact that transcription proceeds in a 5' to 3' fashion along the DNA strand. The promoter and enhancer elements which direct transcription of a linked gene are generally located 5' or upstream of the coding region (enhancer elements can exert their effect even when located 3' of the promoter element and the coding region). Transcription termination and polyadenylation signals are located 3' or downstream of the coding region.

The term "an oligonucleotide having a nucleotide sequence encoding a gene" means a DNA sequence comprising the coding region of a gene or, in other words, the DNA sequence that encodes a gene product. The coding region may be present in either a cDNA or genomic DNA form. Suitable control elements such as enhancers/promoters, splice junctions, polyadenylation signals, etc. may be placed in close proximity to the coding region of the gene if needed to permit proper initiation of transcription and/or correct processing of the primary RNA transcript. Alternatively, the coding region utilized in the expression vectors of the present invention may contain endogenous enhancers/promoters, splice junctions, intervening sequences, polyadenylation signals, etc. or a combination of both endogenous and exogenous control elements.

The term "recombinant oligonucleotide" refers to an oligonucleotide created using molecular biological manipulations, including but not limited to, the ligation of two or more oligonucleotide sequences generated by restriction enzyme digestion of a polynucleotide sequence, the synthesis of oligonucleotides (e.g., the synthesis of primers or oligonucleotides) and the like.

The term "expression vector" as used herein refers to a recombinant DNA molecule containing a desired coding sequence and appropriate nucleic acid sequences necessary for the expression of the operably linked coding sequence in a particular host organism. Nucleic acid sequences necessary for expression in prokaryotes include a promoter, optionally an operator sequence, a ribosome binding site and possibly other sequences. Eukaryotic cells are known to utilize promoters, enhancers, and termination and polyadenylation signals. Once transformed into a suitable host, the vector may replicate and function independently of the host genome, or may, in some instances, integrate into the genome itself. As used herein, "plasmid" and "vector" are sometimes used interchangeably as the plasmid is the most commonly used form of vector at present. However, the invention is intended to include such other forms of expression vectors which serve equivalent functions and which are, or become, known in the art, including but not limited to plasmids, phage particles, or simply potential genomic inserts.

The "host cells" used in the present invention generally are prokaryotic or eukaryotic hosts which contain the expression vector and/or gene of interest. Host cells are transformed or transfected with vectors constructed using recombinant DNA techniques. Such transformed host cells are capable of either replicating vectors encoding the protein variants or expressing the desired protein variant. In the case of vectors which encode the pre- or prepro-form of the protein variant, such variants, when expressed, are typically secreted from the host cell into the host cell medium.

Efficient expression of recombinant DNA sequences in eukaryotic cells requires signals directing the efficient termination and polyadenylation of the resulting transcript. Transcription termination signals are generally found downstream of the polyadenylation signal and are a few hundred nucleotides in length. The term "poly A site" or "poly A sequence" as used herein denotes a DNA sequence which directs both the termination and polyadenylation of the nascent RNA transcript. Efficient polyadenylation of the recombinant transcript is desirable as transcripts lacking a poly A tail are unstable and are rapidly degraded. The poly A signal utilized in an expression vector may be "heterologous" or "endogenous." An endogenous poly A signal is one that is found naturally at the 3' end of the coding region of a given gene in the genome. A heterologous poly A signal is one which is isolated from one gene and placed 3' of another gene. A commonly used heterologous poly A signal is the SV40 poly A signal.

The term "stable transfection" or "stably transfected" refers to the introduction and integration of foreign DNA into the genome of the transfected cell. The term "stable transfectant" refers to a cell which has stably integrated foreign or exogenous DNA into the genomic DNA of the transfected cell.

The terms "selectable marker" or "selectable gene product" as used herein refer to the use of a gene which encodes an enzymatic activity that confers resistance to an antibiotic or drug upon the cell in which the selectable marker is expressed. Selectable markers may be "dominant"; a dominant selectable marker encodes an enzymatic activity which can be detected in any mammalian cell line. Examples of dominant selectable markers include the bacterial aminoglycoside 3' phosphotransferase gene (also referred to as the *neo* gene) which confers resistance to the drug G418 in mammalian cells, the bacterial hygromycin G phosphotransferase (*hyg*) gene which confers resistance to the antibiotic hygromycin and the bacterial xanthine-guanine phosphoribosyl transferase gene (also referred to as the *gpt* gene) which confers the ability to grow in the presence of mycophenolic acid. Other selectable markers are not dominant in that their use must be in conjunction with a cell line that lacks the relevant enzyme activity. Examples of non-dominant selectable markers include the thymidine kinase (*tk*) gene which is used in conjunction with TK⁻ cell lines, the carbamoyl-phosphate synthetase-aspartate transcarbamoylase-dihydroorotase (CAD) gene which is used in conjunction with CAD-deficient cells and the mammalian hypoxanthine-guanine phosphoribosyl transferase *(hprt)* gene which is used in conjunction with HPRT⁻ cell lines. It is noted that some selectable markers can be amplified and therefore can be used as amplifiable markers (*e.g.*, the CAD gene).

The terms "nucleic acid molecule encoding," "DNA sequence encoding," and "DNA encoding" refer to the order or sequence of deoxyribonucleotides along a strand of deoxyribonucleic acid. The order of these deoxyribonucleotides determines the order of amino acids along the polypeptide (protein) chain. The DNA sequence thus codes for the amino acid sequence.

"Homolog" as used herein means a protein (e.g., an enzyme) which has similar catalytic action, structure and/or use as the protein of interest. It is desirable to find a homolog that has a tertiary and/or primary structure similar to the protein of interest as replacement of the epitope in the protein of interest with an analogous segment from the homolog will reduce the disruptiveness of the change. Thus, in most cases, closely homologous proteins provide the most desirable source of epitope substitutions. Alternatively, if possible, it is advantageous to look to human analogs for a given protein. For example, in some embodiments, substituting a specific epitope in one food allergen with a sequence from another food protein that is not allergenic results in the production of a food protein having reduced allergenicity.

As used herein, the term "substantially identical" in the context of two nucleic acids or polypeptides thus typically means that a polynucleotide or polypeptide comprises a sequence that has at least 70% sequence identity, preferably at least 80%, more preferably at least 90%, still more preferably 95%, most preferably 97%, and sometimes as much as 97%, compared to the reference sequence using the known programs such as BLAST, ALIGN, and CLUSTAL using standard parameters (See *e.g.,* Altschul et al., J. Mol. Biol. 215:403-410 [1990]; See *also,* Henikoff et al., Proc. Natl. Acad Sci. USA 89:10915 [1989]; and Karin et al., Proc. Natl Acad. Sci USA 90:5873 [1993]). Software for performing BLAST analyses is publicly available through the National Center for Biotechnology Information. One indication that two polypeptides are substantially identical is that the first polypeptide is immunologically cross-reactive with the second polypeptide. Typically, polypeptides that differ by conservative amino acid substitutions are immunologically cross-reactive. Thus, for example, a polypeptide is substantially identical to a second polypeptide, where the two peptides differ only by a conservative substitution. Another indication that two nucleic acid sequences are substantially identical is that the two molecules hybridize to each other under stringent conditions (*e.g.*, within a range of medium to high stringency).

As used herein, the term "analogous" sequence refers to a sequence that shares particular characteristics with the original protein of interest. Analogous sequences may be determined by ensuring that the replacement amino acids show a similar function, the tertiary structure and/or conserved residues to the amino acids in the protein of interest at or near the epitope. Thus, where the epitope region contains, for example, an alpha-helix or a beta-sheet structure, the replacement amino acids preferably maintain that specific structure.

As used herein, "equivalent residues" refers to proteins that share particular amino acid residues. For example, equivalent resides may be identified by determining homology at the level of tertiary structure for a protein (*e.g.*, a food allergen such as Ber e1) whose tertiary structure has been determined by x-ray crystallography. Equivalent residues are defined as those for which the atomic coordinates of two or more of the main chain atoms of a particular amino acid residue of the protein having putative equivalent residues and the protein of interest (N on N, CA on CA, C on C and O on O) are within 0.13 nm and preferably 0.1 nm after alignment. Alignment is achieved after the best model has been oriented and positioned to give the maximum overlap of atomic coordinates of non-hydrogen protein atoms of the proteins analyzed. The preferred model is the crystallographic model giving the lowest R factor for experimental diffraction data at the highest resolution available, determined using methods known to those skilled in the art of crystallography and protein characterization/analysis.

Some of the residues identified for substitution, insertion or deletion are conserved residues whereas others are not. In the case of residues which are not conserved, the replacement of one or more amino acids is limited to substitutions which produce a variant which has an amino acid sequence that does not correspond to one found in nature. In the case of conserved residues, such replacements should not result in a naturally-occurring sequence.

In some embodiments, the following cassette mutagenesis method finds use in facilitating the construction of the protein variants of the present invention, although other methods may be used. First, the naturally-occurring gene encoding the protein is obtained and sequenced in whole or in part. Then the sequence is scanned for a point at which it is desired to make a mutation (deletion, insertion or substitution) of one or more amino acids in the encoded protein. The sequences flanking this point are evaluated for the presence of restriction sites for replacing a short segment of the gene with an oligonucleotide pool which when expressed will encode various mutants. Such restriction sites are preferably unique sites within the protein gene so as to facilitate the replacement of the gene segment. However, any convenient restriction site which is not overly redundant in the protein gene may be used, provided the gene fragments generated by restriction digestion can be reassembled in proper sequence. If restriction sites are not present at locations within a convenient distance from the selected point (from 10 to 15 nucleotides), such sites are generated by substituting nucleotides in the gene in such a fashion that neither the reading frame nor the amino acids encoded are changed in the final construction. Mutation of the gene in order to change its sequence to conform to the desired sequence is accomplished by M13 primer extension in accord with generally known methods. The task of locating suitable flanking regions and evaluating the needed changes to arrive at two convenient restriction site sequences is made routine by the redundancy of the genetic code, a restriction enzyme map of the gene and the large number of different restriction enzymes. Note that if a convenient flanking restriction site is available, the above method need be used only in connection with the flanking region which does not contain a site.

Once the naturally-occurring DNA or synthetic DNA is cloned, the restriction sites flanking the positions to be mutated are digested with the cognate restriction enzymes and a plurality of end termini-complementary oligonucleotide cassettes are ligated into the gene. The mutagenesis is simplified by this method because all of the oligonucleotides can be synthesized so as to have the same restriction sites, and no synthetic linkers are necessary to create the restriction sites.

### Description of the Invention

The present invention provides methods and composition concerning the identification and modification of allergenic proteins. More particularly, the present invention provides methods for the modification of epitopes of the allergenic Ber e1 protein, as well as its epitopes of Seq. IDs 4,6 and 7. In particular, the present invention provides CD4+ T-cell epitopes of the 2S globulin storage protein of the Brazil nut (*Bertholletia excelsa*). The allergen associated with this protein is designated "Ber e1" (*See e.g.*, Nordlee et al., New Eng. J. Med., 334:688-692 [1996]). The polynucleotide sequence and protein sequence of this protein are provided in Altenbach et al., Plant Mol. Biol., 8:239-250 (1987); Gander et al., Plant Mol. Biol. 16:437-448 (1991); and Ampe et al., Eur. J. Biochem. (FEBS) 159:597-604 (1986).

In addition, the present disclosure provides the CD4+ T-cell epitopes of two known *Bacillus thuringiensis* Cry proteins. *Bacillus thuringiensis* (Bt) is a bacterial species that produces a crystal protein that is a protoxin. This protoxin is also referred to as a delta-endotoxin. The protoxin is converted into a toxin upon ingestion by insect larvae. The toxin is particularly effective against lepidopteran, coleopteran and dipteran insects that create major problems in agriculture and forestry. Many Bt proteins have been isolated and sequenced. These include but are not limited to Cry1Aa, Cry1Ab, Cry1Ac, CryIC, CryIE, CryICb, CryIF, Cry3Aa, Cry5 and others. Various classifications exist for the Cry proteins. For example, Hofte and Whiteley, Microbiol. Rev., 53:242-255 (1989) present one early example of a classification system, but many new Cry proteins have been discovered and isolated since this classification was proposed.

A number of genes have been constructed which include the coding sequence for Bt Cry proteins. In particular, corn and potato cells have been transformed with constructs including Bt cry genes or genes encoding a protein having substantially the insect toxicity properties of the Bt toxin, in order to increase the productivity of these crops. In contrast to Brazil nut which may cause a severe allergenic response in certain humans, the Cry proteins which have been incorporated into animal and human crop plants is not known to cause any significant allergenic response. However, there is concern that genetically-modified foods will result in allerg ic responses in humans and other animals due to the introduction of proteins not previously in the food supply. Thus, means to identify and/or modify food proteins that may be allergenic find use in assessing the safety of genetically-modified foods.

Indeed, the present disclosure provides means to determine the T-cell epitopes in such proteins commonly used in transgenic food crops. Cry1Ab is one of the most common Cry proteins and shares at least 80% amino acid sequence homology with CryIAa and CryIAc (*See e.g.*, Hofte et al., Eur. J. Biochem. 161:273 [1986]). Thus, the present disclosure also provides means to identify and/or modify potential T-cell epitopes in Cry3Aa (See e.g., Hofte et al., Nucl. Acids Res., 15: 7183 [1987]; and Sekar et al., Proc. Natl. Acad. Sci. USA 847036-7040 [1987]).

Epitopes may be identified using an assay system that identifies epitopes and non-epitopes. Differentiated dendritic cells are combined with human CD4+ and/or CD8+ T-cells and with a peptide of interest (*e.g.*, a food allergen or putative food allergen). More specifically, a food protein having reduced allergenicity can be provided. In this assay system, a T-cell epitope is recognized by means comprising the steps of: (a) obtaining from a single blood source a solution of dendritic cells and a solution of naïve CD4+ T-cells; (b) promoting differentiation in said solution of dendritic cells; (c) combining said solution of differentiated dendritic cells and said CD4+ T-cells with a peptide of interest; (d) measuring the proliferation of the T-cells in said step (c).

A series of peptide oligomers that correspond to all or parts of the test protein (*e.g.*, food allergen) may be prepared. For example, a peptide library is produced covering the relevant portion or all of the test protein. The manner of producing the peptides is to introduce overlap into the peptide library, for example, producing a first peptide corresponds to amino acid sequence 1-15 of the test protein, a second peptide corresponds to amino acid sequence 4-18 of the test protein, a third peptide corresponds to amino acid sequence 7-21 of the test protein, a fourth peptide corresponds to amino acid sequence 10-24 of the test protein etc., until representative peptides corresponding to the entire molecule are created. By analyzing each of the peptides individually in the assay provided herein, it is possible to precisely identify the location of epitopes recognized by T-cells. In the example above, the greater reaction of one specific peptide than its neighbors' facilitates identification of the epitope anchor region to within three amino acids. After determining the location of these epitopes, it is possible to alter the amino acids within each epitope until the peptide produces a different T-cell response from that of the original protein. Moreover, food proteins may be identified herein which have desired low T-cell epitope potency which may be used in their naturally occurring forms.

Various *in vitro* and *in vivo* assays known in the art find use in the ascertainment of the reduced immunogenic response of variant proteins. Suitable assay systems include, but are not limited to *in vivo* assays such as HLA-DR3/DQ2 mouse T cell responses and *in vitro* assays such as human peripheral blood mononuclear cells (PBMC) assays (See, Herman et al., J. Immunol., 163:6275-6282; Sonderstrup et al., Immunol. Revi,172: 335-343 [1999]; Taneja and David, Immunol. Rev., 169:67-79 [1999]; and Grusby et al., Proc. Natl. Acad. Sci. USA 90:3913-3917 [1993]).

One or more CD4+ T-cell epitopes were determined for each of the Ber e1 protein, Cry 1Ab protein and Cry3Aa protein.

The identified T-cell epitope of the Ber e1 protein of Seq. ID 4, 6, or 7 is modified to produce variant(s) with reduced allergenic capacity. The present disclosure provides means to reduce the allergenic response of a Ber e1 protein comprising obtaining a precursor Ber e1 protein and obtaining a variant of the precursor Ber e1 protein, wherein the variant has at least one T-cell epitope of the precursor protein and wherein the variant exhibits an altered allergenic response which differs from the allergenic response of the precursor Ber e1 protein.

The epitopes identified herein can be used to elicit an immune response (*e.g.*, where it is desired to raise antibodies against a protein of interest). Such antibodies can be used, for example, to screen for other food proteins that include one or more of the epitope regions, or regions highly homologous thereto. Thus, the methods of the present invention find use in the development of additional systems for use in assessing food allergies.

Additionally, the epitopic fragments herein can be used in the detection of antigen presenting cells having MHC molecules capable of binding and displaying such fragments. For example, the epitopic fragments can include a detectable label (*e.g.*, radiolabel). The labeled fragments can then be incubated with cells of interest, and then cells that bind (or display) the labeled fragments can be detected.

In additional embodiments, the present disclosure provides means to determine the HLA associations with reactivity to specific food allergen epitopes. For example in experiments conducted during the development of the present invention, the HLA-DR and DQ types of donors who responded to peptide #4 (light chain amino acids 10-24 of Ber e1) and peptide #30 (heavy chain amino acids 40-55 of Ber e1) were analyzed for the presence of any statistically significant enrichments. HLA-DQ2 was found to be significantly associated with a non-response to peptide #4 (p = 0.05, relative risk = 0.29). There was a positive association between the presence of HLA-DR15(2) and a response to peptide #20 (p = 0.04, relative risk = 3.0). The strongest association found was a positive association between a response to peptide #12 and the presence of HLA-DR13 (p - 0.01, relative risk = 4.44). Thus, it is contemplated that the present invention will find use in identifying individuals who are potentially allergic to any food allergen of interest.

### EXPERIMENTAL

The following examples are provided in order to demonstrate and further illustrate certain preferred embodiments and aspects of the present invention, or disclosure and are not to be construed as limiting the scope thereof.

In the experimental disclosure which follows, the following abbreviations apply: °C (degrees Centigrade); rpm (revolutions per minute); xg (times gravity); IgG (immunoglobulin G); IM (intramuscular); IP (intraperitoneal); IV (intravenous or intravascular); SC (subcutaneous); H₂O (water); HCl (hydrochloric acid); aa (amino acid); bp (base pair); kb (kilobase pair); kD (kilodaltons); gm (grams); µg (micrograms); mg (milligrams); ng (nanograms); µl (microliters); ml (milliliters); mm (millimeters); nm (nanometers); µm (micrometer); M (molar); mM (millimolar); µM (micromolar); U (units); V (volts); MW (molecular weight); sec (seconds); min(s) (minute/minutes); hr(s) (hour/hours); MgCl₂ (magnesium chloride); NaCl (sodium chloride); OD₂₈₀ (optical density at 280 nm); OD₆₀₀ (optical density at 600 nm); PAGE (polyacrylamide gel electrophoresis); PBS (phosphate buffered saline [150 mM NaCl, 10 mM sodium phosphate buffer, pH 7.2]); PCR (polymerase chain reaction); RT-PCR (reverse transcription PCR); SDS (sodium dodecyl sulfate); Tris (tris(hydroxymethyl)aminomethane); w/v (weight to volume); v/v (volume to volume); Amersham (Amersham Life Science, Inc. Arlington Heights, IL); ICN (ICN Pharmaceuticals, Inc., Costa Mesa, CA); Amicon (Amicon, Inc., Beverly, MA); Clontech (CLONTECH Laboratories, Palo Alto, CA); Difco (Difco Laboratories, Detroit, MI); GIBCO BRL or Gibco BRL (Life Technologies, Inc., Gaithersburg, MD); Invitrogen (Invitrogen Corp., San Diego, CA); Endogen (Endogen, Wobum, MA); CytoVax (CytoVax, Edmonton, Canada); Wyeth-Ayerst (Wyeth-Ayerst, Philadelphia, PA); NEN (NEN Life Science Products, Boston, MA); Wallace Oy (Wallace Oy, Turku, Finland); Pharma AS (Pharma AS, Oslo, Norway); Dynal (Dynal, Oslo, Norway); Bio-Synthesis (Bio-Synthesis, Lewisville, TX); ATCC (American Type Culture Collection, Rockville, MD); Gibco/BRL (Gibco/BRL, Grand Island , NY); Sigma (Sigma Chemical Co., St. Louis, MO); Pharmacia (Pharmacia Biotech, Pisacataway, NJ); and Stratagene (Stratagene, La Jolla, CA).

### EXAMPLE 1 (not part of the invention)

### Assay for the-identification of Peptide T-Cell Epitopes in Brazil Nut Seed Storage Protein and in Bacillus thuringiensis Cry Protein Using Human T-Cells

Fresh human peripheral blood cells were collected from 48, 47 or 92 humans of unknown exposure status to food proteins and were tested for the determination of antigenic epitopes in Cry1Ab, Cry3Aa, and Ber e1. Peripheral mononuclear blood cells (stored at room temperature, no older than 24 hours) were prepared for use as described herein. Approximately 30 mls of a solution of buffy coat preparation from one unit of whole blood was brought to 50 ml with Dulbecco's phosphate buffered solution (DPBS) and split into two tubes. The samples were underlaid with 12.5 ml of room temperature lymphoprep density separation media (Nycomed density 1.077 g/ml). The tubes were centrifuged for thirty minutes at 600 xg. The interface of the two phases was collected, pooled and washed in DPBS. The cell density of the resultant solution was measured by hemocytometer, as known in the art. Cell viability was measured by trypan blue exclusion, as known in the art.

From the resulting solution, a differentiated dendritic cell culture was prepared from the peripheral blood mononuclear cell sample having a density of 10⁸ cells per 75 ml culture flask in a solution as follows:
(1) 50 ml of serum free AIM V medium (Gibco) was supplemented with a 1:100 dilution beta-mercaptoethanol (Gibco). The flasks were laid flat for two hours at 37°C in 5% CO2 to allow adherence of monocytes to the flask wall;
(2) Differentiation of the monocyte cells to dendritic cells was performed as follows: nonadherent cells were removed and the resultant adherent cells (monocytes) combined with 30 ml of AIM V, 800 units/ml of GM-CSF (Endogen) and 500 units/ml of IL-4 (Endogen); the resulting mixture was cultured for 5 days under conditions at 37°C in 5% CO₂. After five days, the cytokine TNFalpha (Endogen) was added to 0.2 units/ml, and the cytokine IL-1a (Endogen) was added to a final concentration of 50 units/ml and the mixture incubated at 37°C in 5% CO₂ for two more days;
(3) On the seventh day, mitomycin C was added to a concentration of 50 microgram/ml was added to stop growth of the now differentiated dendritic cell culture. The solution was incubated for 60 minutes at 37°C in 5% CO₂. Dendritic cells were collected by gently scraping the adherent cells off the bottom of the flask with a cell scraper. Adherent and non-adherent cells were then centrifuged at 600xg for 5 minutes, washed in DPBS and counted; and
(4) The prepared dendritic cells were placed into a 96 well round bottom array plate at 2x10⁴ cells/well in 100 microliter total volume of AIM V medium.

CD4+ T cells were prepared from frozen aliquots of the peripheral blood cell samples used to prepare the dendritic cells. These T-cells were prepared using the human CD4+ Cellect Kit (Biotex) as per the manufacturer's instructions, but with the following modifications: the aliquots were thawed and washed such that approximately 10⁸ cells were applied to each Cellect column. First, the cells were suspended in 4 ml DPBS and 1 ml of the Cell reagent from the Cellect Kit, with the solution being maintained at room temperature for 20 minutes. Following this 20 minute incubation, the cell solution was centrifuged for five minutes at 600xg at room temperature and the pellet was resuspended in 2 ml of DPBS and applied to the Cellect columns. The effluent from the columns was collected in 2% human serum in DPBS. The resultant CD4+ cell solution was centrifuged, resuspended in AIMV medium and the cell density determined. The CD4+ T-cell suspension was adjusted to provide 2x10⁶ cells/ml in AIM V medium to facilitate efficient manipulation of the 96 well plate used in the assay system.

Synthetic 15-mer peptides corresponding to the sequence of the test protein are resuspended in DMSO to create a stock solution at a concentration of 1 mM. Peptide antigen was prepared for use in the assay by diluting the 1 mM stock into AIM V media at a ratio of 1:10. Then, 10 µl of the diluted peptide solution was added to each well of the 96 well plate containing the differentiated dendritic cells. Finally, 100 microliters of the diluted CD4+ T-cell solution as prepared above was added to each well. Useful controls include diluted DMSO blanks, and tetanus toxoid positive controls.

The final concentrations in each well, at 210 microliter total volume are as follows:
2x10⁴ CD4+ cells
2x10⁵ dendritic cells (R:S of 10:1)
5 µM peptide

### Example 2

### Identification of T-Cell Epitopes in Ber e1 from Bertholletia excelsa (part of the invention) and from Cry 1Ab and Cry3A from Bacillus thuringiensis (not part of the invention).

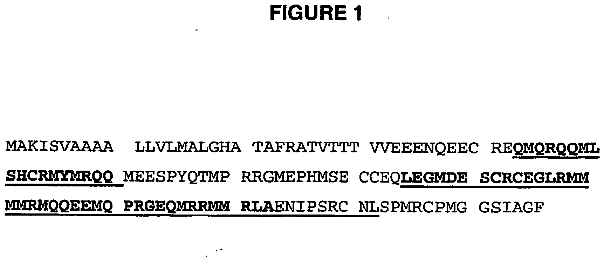

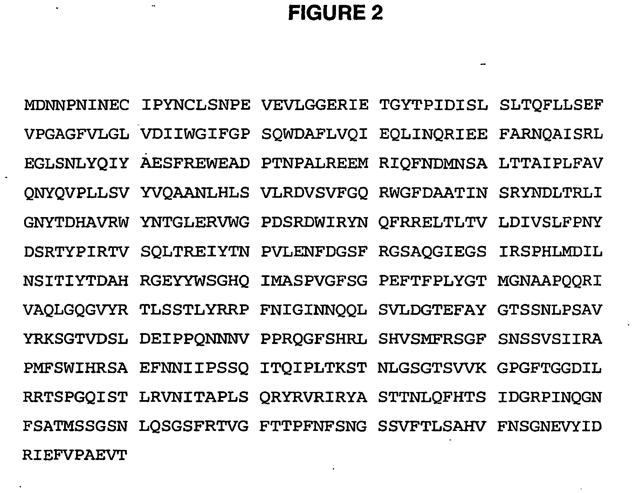

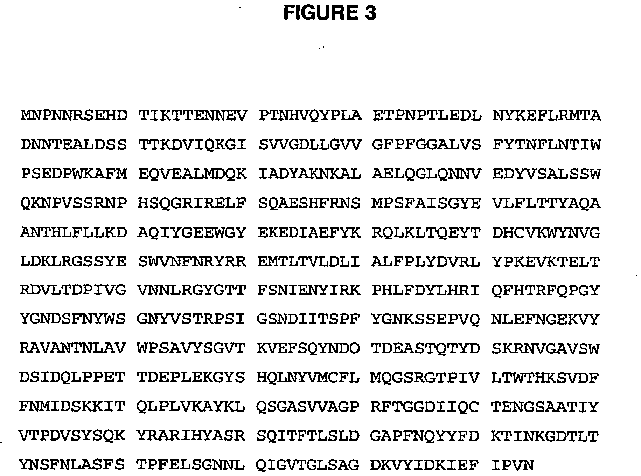

Peptides for use in the assay described in Example 1 were prepared based on the Ber e1 protein of *Bertholletia excelsa,* as well as Cry 1Ab, and Cry3Aa from *Bacillus thuringiensis* amino acid sequences (See, Figures 1, 2, and 3). Peptide antigens were designed essentially the same for each protein. From the full length amino acid sequence of the protein of interest starting with the first amino acid residue as provided in Figures 1 - 3, 15mers were synthetically prepared, with each 15mer overlapping with the previous and the subsequent 15mer except for three residues. All tests were performed at least in duplicate. All tests reported displayed robust positive control responses to the antigen tetanus toxoid. Responses were averaged within each experiment, then normalized to the baseline response. A positive event was recorded if the response was at least 2.95 times the baseline response.

The results are illustrated in Figures 4, 5 and 6. Results for all donors were compiled. The percentage of the donor pool that responded with a baseline-normalized response of 2.95 or better was calculated for each peptide. The background response rate for each peptide set corresponding to each protein tested was calculated by averaging all the percent responses to all the peptides within a set. The background response for the Ber e 1 peptide set was determined to be 4.27%. As indicated above, a "major epitope" is a peptide sequence that induces responses in a percentage of the donor pool equal to three times the background rate. For the Ber e 1 dataset, this is equal to a value of 12.81%. As shown in Figure 4, only the response to peptide #4 reached the three-fold cut-off (peptide #4 value = 18.48%). Weak epitopes are designated by responses that reach or exceed twice the background rate (i.e., 8.54% for the Ber e 1 dataset). Two peptides (#12 and #20) were identified as minor epitopes. Peptide # 16 is prominent in the dataset, but does not reach any epitope designation. These peptides correspond to the following sequences:

| | |
|---|---|
| Peptide 4 = RQQMLSHCRMYMRQQ | (SEQ ID NO: 7) |
| Peptide 12 = LEGMDESCRCEGLRM | (SEQ ID NO: 4) |
| Peptide 16 = LRMMMMRMQQEEMQP | (SEQ ID NO: 5) (not part of the invention) |
| Peptide 20 = MQPRGEQMRRMMRLA | (SEQ ID NO: 6) |

As indicated in Figure 6, only 1 major T-cell epitope was determined from Cry1Ab. The background value was calculated for the dataset in the absence of the zero values. The major epitope (Peptide 145; VSMFRSGFSNSSVSI [SEQ ID NO: 8]) in this protein reached three times this background rate. However, multiple weak epitopes for Cry1Ab were observed, including:

| | |
|---|---|
| Peptide 26 = FLVQIEQLINQRIEE | (SEQ ID NO: 9) |
| Peptide 35 = LSNLYQIYAESFREW | (SEQ ID NO: 10) |
| Peptide 124 = PFNIGINNQQLSVLD | (SEQ ID NO: 11) |
| Peptide 137 = SLDEIPPQNNNVPPR | (SEQ ID NO: 12) |
| Peptide 174 = SQRYRVRIRYASTTN | (SEQ ID NO: 13) |

As indicated in Figure 5, no major T-cell epitopes were determined for Cry3Aa. However, two weak epitopes were observed:

| | |
|---|---|
| Peptide 40 = DQKIADYAKNKALAE | (SEQ ID NO: 14) |
| Peptide 47 = NVEDYVSALSSWQKN | (SEQ ID NO: 15) |

### Example 3

### Determination of Variants with Altered Allergenicity Residues Within an Epitope

Peptide variants based on peptide # 12, 16 and 20 are tested in the mapping assay described above, with 20 or more community donor blood samples. For each of the peptide variants, three amino acid offset 15-mers are constructed to cover the entire region of the proposed change. The parent peptides in the set may be analyzed by mass spectrometry, as desired. In addition, crystal structure analysis is sometimes performed, in order to guide the selection of amino acid variants, as this helps preserve the function and structure of the molecule.

An alanine scan is performed for each peptide. In addition, variant peptides corresponding to the major and weak epitopes are generated using methods known in the art. The assay is performed as described in Example 3, utilizing the variant peptides on a set of donor samples. Proliferative responses are collated, and the results compared with the results obtained with the original peptide sequences. In some experiments, additional testing is used to confirm the statistical relevance of the results. Based on the known structure of the food allergens, a number of alternative sequences in the peptides corresponding to the major and weak epitopes (as desired) are selected, such that it they are predicted to have little effect on the protein structure, but are different enough to potentially affect the recognition of the epitope by the immune system. These modified epitopes then find use in modifying food allergens such that the food allergens are hypoallergenic.

From the above, it is clear that the present invention provides methods and compositions concerning the identification of T-cell epitopes in food allergen proteins and the production of peptides which when incorporated into the food allergen sequence, are no longer capable of initiating the CD4⁺ T-cell response. In particular, the present invention provides means and compositions suitable for reducing the allergenicity of food proteins.

## Claims

1. A method for reducing the allergenicity of Ber e1 protein comprising obtaining a precursor Ber e1 protein and modifying a T cell epitope within said precursor Ber e1 protein to provide a variant Ber e1 protein, whereby the variant Ber e1 protein exhibits a reduced allergenic response compared to the precursor Ber e1 protein, wherein said T cell epitope is SEQ ID NO: 4, SEQ ID NO: 6 or SEQ ID NO: 7.

2. A method according to claim 1 comprising the steps of:
a) modifying a T cell epitope region of said Ber e1 protein to produce a variant Ber e1 protein;
b) contacting a plurality of amino acid fragments of said variant Ber e1 with a solution comprising naïve human CD4+ T-cells and dendritic cells, wherein said dendritic cells have been differentiated in vitro
c) measuring the ability of said amino acid fragments of said variant Ber e1 protein to stimulate proliferation of said naïve human CD4+ T-cells; and
d) comparing the ability of said amino fragments of said variant Ber e1 protein to stimulate the proliferation of naïve human CD4+T-cells with the ability of corresponding amino acid fragments of said Ber e1 protein to stimulate the proliferation of naïve human CD4+ T-cells;
wherein said T cell epitope region is SEQ ID NO: 4, SEQ ID NO: 6 or SEQ ID NO: 7.

3. The method of claim 2, wherein said dendritic cells and said CD4+ T-cells in said first and second solutions are obtained from a single blood source.

4. The method of claim 1 or claim 2, further comprising the step of producing an expression vector comprising a nucleic acid sequence encoding said variant Ber e1 protein having reduced allergenicity.

5. The method of claim 4, further comprising transforming at least one host cell with said expression vector.

6. The method of claim 5, further comprising the steps of cultivating said at least one host cell in a culture medium under conditions that promote the expression of said variant Ber e1 protein having reduced allergenicity and recovering said variant Ber e1 protein having reduced allergenicity from said cell or said culture medium.

7. A peptide consisting of the sequence of SEQ ID NO: 4, SEQ ID NO: 6 or SEO ID NO: 7.

## Patentansprüche

1. Verfahren zur Reduktion der Allergenität von Ber-e1-Protein, umfassend das Erhalten eines Ber-e1-Vorläuferproteins und das Modifizieren eines T-Zell-Epitops in dem Ber-e1-Vorläuferprotein, um eine Ber-e1-Proteinvariante bereitzustellen, wobei die Ber-e1-Proteinvariante eine im Vergleich mit dem Ber-e1-Vorläuferprotein reduzierte allergene Reaktion an den Tag legt, worin das T-Zell-Epitop Seq.-ID Nr. 4, Seq.-ID Nr. 6 oder Seq.-ID Nr. 7 ist.

2. Verfahren nach Anspruch 1, umfassend die folgenden Schritte:
a) das Modifizieren einer T-Zell-Epitopregion des Ber-e1-Proteins, um eine Ber-e1-Proteinvariante zu erzeugen;
b) das Kontaktieren mehrerer Aminosäurefragmente der Ber-e1-Variante mit einer Lösung, die naive menschliche CD4+-T-Zellen und dendritische Zellen umfasst, worin die dendritischen Zellen in vitro differenziert wurden,
c) das Messen der Fähigkeit der Aminosäurefragmente der Ber-e1-Proteinvariante, um Proliferation der naiven menschlichen CD4+-T-Zellen zu stimulieren, und
d) das Vergleichen der Fähigkeit der Aminosäurefragmente der Ber-e1-Proteinvariante, die Proliferation naiver menschlicher CD4+-T-Zellen zu stimulieren, mit der Fähigkeit der entsprechenden Aminosäurefragmente des Ber-e1-Proteins, die Proliferation naiver menschlicher CD4+-T-Zellen zu stimulieren,
worin die T-Zell-Epitopregion Seq.-ID Nr. 4, Seq.-ID Nr. 6 oder Seq.-ID Nr. 7 ist.

3. Verfahren nach Anspruch 2, worin die dendritischen Zellen und die CD4+-T-Zellen in der ersten und der zweiten Lösung aus einer einzigen Blutquelle erhalten wurden.

4. Verfahren nach Anspruch 1 oder Anspruch 2, weiters umfassend den Schritt des Herstellens eines Expressionsvektors, der eine für die Ber-e1-Proteinvariante mit reduzierter Allergenität kodierende Nucleinsäuresequenz umfasst.

5. Verfahren nach Anspruch 4, weiters umfassend das Transformieren zumindest einer Wirtszelle mit dem Expressionsvektor.

6. Verfahren nach Anspruch 5, weiters umfassend den Schritt des Kultivierens der zumindest einen Wirtszelle in einem Nährmedium unter Bedingungen, die die Expression der Ber-e1-Proteinvariante mit reduzierter Allergenität fördern, und den des Gewinnens der Ber-e1-Proteinvariante mit reduzierter Allergenität aus der Zelle oder dem Nährmedium.

7. Peptid, bestehend aus der Sequenz von Seq.-ID Nr. 4, Seq.-ID Nr. 6 oder Seq.-ID Nr. 7.

## Revendications

1. Méthode pour réduire l'allergénicité de la protéine Ber e1 comprenant l'obtention d'une protéine Ber e1 précurseur et la modification d'un épitope de cellule T dans ladite protéine Ber e 1 précurseur pour obtenir une protéine Ber e1 variante, moyennant quoi la protéine Ber e1 variante présente une réponse allergénique réduite en comparaison avec la protéine Ber e1 précurseur, où ledit épitope de cellule T est SEQ ID NO:4, SEQ ID NO:6 ou SEQ ID NO: 7.

2. Méthode selon la revendication 1, comprenant les étapes de:
a) modifier une région d'épitope de cellule T de ladite protéine Ber e1 pour produire une protéine Ber e1 variante;
b) mettre en contact une pluralité de fragments d'acide aminé de ladite Ber e1 variante avec une solution comprenant des cellules T CD4+ humaines naïves et des cellules dendritiques, où lesdites cellules dendritiques ont été différenciées in vitro;
c) mesurer l'aptitude desdits fragments d'acide aminé de ladite protéine Ber e1 variante pour stimuler la prolifération desdites cellules T CD4+ humaines naïves; et
d) comparer l'aptitude desdits fragments d'acide aminé de ladite protéine Ber e1 variante pour stimuler la prolifération des cellules T CD4+ humaines naïves avec l'aptitude de fragments d'acide aminé correspondants de ladite protéine Ber e1 pour stimuler la prolifération des cellules T CD4+ humaines naïves;
où ladite région d'épitope de cellule T est la SEQ ID NO:4, SEQ ID NO:6 ou SEQ ID NO:7.

3. Méthode selon la revendication 2, où lesdites cellules dendritiques et lesdites cellules T CD4+ dans lesdites première et deuxième solutions sont obtenues à partir d'une seule source de sang.

4. Méthode selon la revendication 1 ou la revendication 2, comprenant en outre l'étape consistant à produire un vecteur d'expression comprenant une séquence d'acides nucléiques codant pour ladite protéine Ber e1 variante ayant une allergénicité réduite.

5. Méthode selon la revendication 4, comprenant en outre la transformation d'au moins une cellule hôte par ledit vecteur d'expression.

6. Méthode selon la revendication 5, comprenant en outre les étapes consistant à cultiver au moins une cellule hôte dans un milieu de culture sous des conditions qui encouragent l'expression de ladite protéine Ber e1 variante ayant une allergénicité réduite et récupérant ladite protéine Ber e1 variante ayant une allergénicité réduite de ladite cellule ou dudit milieu de culture.

7. Peptide constitué de la séquence de SEQ ID NO:4, SEQ ID NO:6 ou SEQ ID NO:7.
